# EUROPEAN PATENT SPECIFICATION

(11) **EP 0 659 080 B1**
(45) Date of publication and mention of the grant of the patent: **11.07.2001**
(21) Application number: 93921500.0
(22) Date of filing: 13.09.1993
(51) Int. Cl.: A61K 31/56, A61K 31/20

(54) **METHOD FOR THE TREATMENT OF HAIR LOSS**
VERFAHREN ZUR BEHANDLUNG VON HAARAUSFALL
PROCEDE UTILE POUR TRAITER LA CHUTE DES CHEVEUX

(30) Priority: 11.09.1992 US 943853
(43) Date of publication of application: 28.06.1995
(73) Proprietor: BROWN, Sandra, Southfield, MI 48034 (US)
(72) Inventor: BROWN, Sandra, Southfield, MI 48034 (US)
(74) Representative: West, Alan Harry
(86) International application number: US9308575
(87) International publication number: WO9406434

(56) References cited:
- EP-A- 0 415 766
- Journal of the American Academy of Dermatology, Vol. 21, No. 2, Part 1, issued August 1989, LESNICK et al., "Topical All-Trans-Retinoic Acid Prevents Corticosteroid-Induced Skin Atrophy without Abrogating the Anti-Inflammatory Effect", pages 186-190, entire document.
- Journal of the American Academy of Dermatology, Vol. 15, No. 4, Part 2, issued December 1986, BAZZANO et al., "Topical Tretinoin for Hair Growth Promotion", pages 880-883, entire document.

## Description

The present invention relates to a cosmetical method for hair loss, and more particularly, to a method using tretinoin and hydrocortisone.

Restoration of human hair has been attempted for centuries. In many cases, hair loss is merely covered by wigs or toupees. Many medical treatments have been attempted over the years; however, up until now, no treatment has been found which satisfactorily stimulates hair growth for a wide variety of cases, including alopecia.

Each hair extends from a tube-like depression called a hair follicle. The hair follicle extends from the surface of the skin into the dermis and may pass into the subcutaneous layer. At the base of the follicle is a group of epidermal cells which receive nourishment from blood vessels that occur in a projection of connective tissue at the base of the follicle.

As the epidermal cells divide and grow, older cells are pushed toward the surface. The cells that move upward and away from the nutrient supply become keratinized and die. Thus, hair is dead keratin, just like scale, and is formed at a predetermined rate.

The normal rate of growth of hair is 1 cm per month. Each hair follicle goes through a cycle of a growth stage (anagen hair), and an involution or resting stage (telogen hair). The anagen stage lasts about three years, while the telogen stage lasts only about three months. Once the hair follicle reaches the telogen stage, the hair falls out. Eventually, the hair follicle regenerates into the anagen stage and new hair is produced.

The cycle of hair activity for hair follicles is independent for each hair follicle. However, when the hair follicles fail to regenerate hair, baldness results.

Many causes of hair loss are known. Exposure to chemotherapy, XRT, and exposure to toxic chemicals can cause anagen hair loss. Hormonal imbalances, stress, nutritional deficiency, and usage of many drugs can cause telogen effluvium. Alopecia areata and androgenetic alopecia are caused by genetics.

It is known that the hair follicle is an immune-privileged organ, and it has been postulated that hair growth may be regulated by the immune system (Frusgate et al, Journal of Investigative Dermatology, 97: 417-420, 1991). Thus, in order to restore hair, it is necessary to treat any underlying causes of the hair loss, such as disease, stress, hormonal imbalance, or nutritional deficiency. It is known, as disclosed in Olson, Alopecia Evaluation, Primary Care 1989: 16 (3), p. 765-787, to treat hair loss by making an evaluation of the patient, including patient history, physical exam, and lab studies, treating any treatable underlying causes of hair loss, and treating alopecia with topical minoxidil and antiandrogens. However, treatment with minoxidil has many undesirable side effects and hair growth occurs only as long as the minoxidil is being used. Thus, it is desirable for restoring hair loss which acts to overcome the causes of alopecia and permits hair follicles to grow hair without continuous external stimulation, such as by minoxidil.

It is known to use various commercial shampoo preparations to strengthen the hair. These shampoos are typically protein and effect only dead keratin, not the hair follicle, and therefore cannot prevent hair loss.

The present method for hair restoration produces hair growth in virtually all cases. The rate of hair growth is greater, than that in previously known methods.

Following a treatment of underlying causes of hair loss, an application of hydrocortisone and tretinoin is made to the patient according to the invention. In the preferred embodiment, the hydrocortisone and tretinoin are administered in a composition applied topically to the scalp. Next, Biotin is administered to the patient. Additionally, the hair may b shampooed with a protein shampoo. The cosmetical method has been found to effectively restore hair growth even after discontinuance of use of the compound.

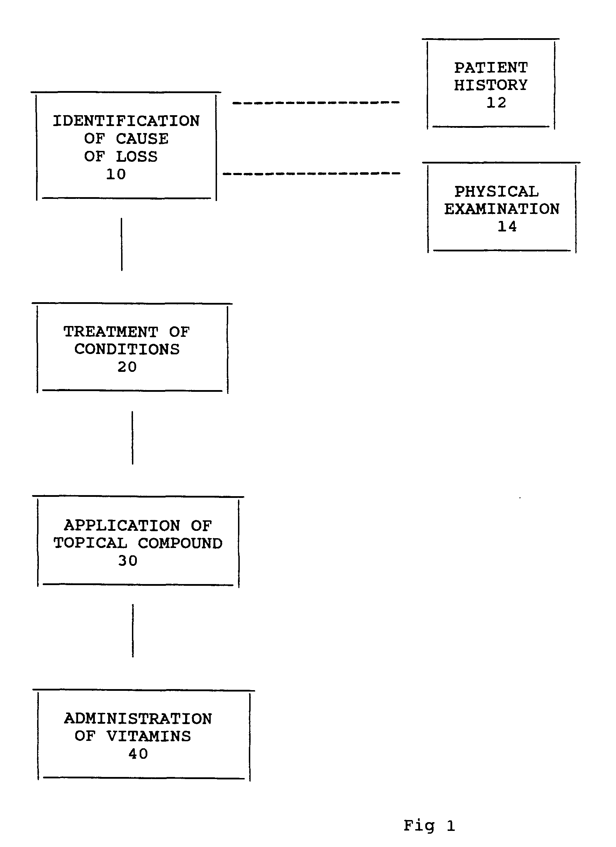

Fig. 1 is a block diagram of the treatment including the cosmetical method according to the invention.

Disclosed herein is a cosmetical method of promoting hair growth using corticosteroids and tretinoin. As best shown in Fig. 1, after identifying the underlying cause of hair loss 10 which includes making a complete patient history 12; internal physical examination 14, external examination of the scalp and hair 16; and treatment of any underlying cause of hair loss 20; tretinoin and hydrocortisone are applied to the scalp of the patient 30; and B complex vitamins are to be administered to stimulate growth 40. In some cases, shampooing with a protein shampoo is performed.

After all treatable causes of hair loss are identified and treatment begun, the patient undergoes the cosmetical method of the invention by being treated with an anti-inflammatory agent, such as a topical corticosteroid and tretinoin. As set forth below, the preferred embodiment is in the form of a topical gel. Anti-inflammatory drugs such as hydrocortisone are known to help promote hair growth by stimulating the hair follicles. However, hydrocortisone is a steroid and has a tendency to thin the dermis.

Tretinoin (also known as all-trans-retinoic acid) is also administered, however, one of the well-known side effects of tretinoin is that it acts to thicken the skin. Thus, the side effects of the combination of the hydrocortisone with tretinoin acts to cancel the negative side effects of each other while producing a synergistic amount of hair. The hydrocortisone and tretinoin may be applied separately or together in the preferred embodiment as a topical preparation in the form of a gel.

### EXAMPLE 1

In example 1, hydrocortisone was injected into the area of the scalp of the patient having hair loss. Hydrocortisone liquid, which is purchased in a concentration of 25 grams per milliliter was diluted with propylene glycol to a concentration of 2.5%. The hairless area of the scalp was delineated into zones of approximately one square inch. An injection of a maximal of 0.1 ml (0.1cc) of the diluted liquid was made in each zone. After the injection, a topical application of tretinoin was made twice a day. Retin A is available from Ortho Pharmaceutical Corp. of Raritan, New Jersey. The tretinoin was obtained in a gel having a concentration of 0.025%. The gel was applied as a thin coat on the hairless area of the scalp. The gel was kept as thin as possible to avoid residue which flakes after drying.

### Results

Four days after initial treatment, new hair growth was observed on a patient who had been bald in that area for twenty years. A second injection of hydrocortisone was made after two weeks and tretinoin gel was applied as before. After a month, the patient had thicker hair in the area that was treated than on the rest of the head. As a result, treatment with the gel was reduced to once a day and hair growth continued. After six weeks, the patient had normal hair growth in the area treated.

### EXAMPLE 2

In example 2, a topical gel was formed having hydrocortisone and tretinoin as main active ingredients. The gel was formed of a 2.5% solution of hydrocortisone, 2.5% solution of tretinoin (Retin A) and 95 parts of propylene glycol. The propylene glycol is a dilutant and maintains the compound in a gel. The percentage strength of hydrocortisone and tretinoin were chosen because they are commercially available in these strengths. It is believed that treatment will be successful with other proportions of the active ingredients. The gel was applied to the affected scalp once a day. Since any excess gel will dry on the scalp and flake, the gel was applied in as thin a coat as possible.

Outstanding results, including hair growth within one week of applying the compound, were observed. It is believed that the hydrocortisone acts to suppress the immune system which acts to suppress hair growth when fighting certain conditions.

In the preferred embodiment of the compound, a gel is formed of equal parts each having a concentration of 0.05% of a long acting hydrocortisone such as Betamethasone Dipropionate made by Lemmor Laboratories of Sellersville, PA and trans-retinoic acid in a powdered form produced by Paddock Laboratories are mixed with propylene glycol.

In addition to treating the patient with the gel, the method also includes administration of Biotin, which is available from Nature's Plus. The Biotin is administered in the amount of 2000 mg per day. Additionally, 100 mg of B complex vitamins may be administered daily.

Additional treatment may be given depending upon the condition of the hair. A shampoo such as Peppar® may be administered to the scalp. Peppar® has protein and is known to stimulate circulation of the hair follicles and is made by Palm Beach Beauty Products. An RJ solution may be administered to thicken the hair.

It is understood, of course, that the invention is not limited to the particular embodiments described herein. For instance, it is within the contemplation of the invention to use any corticosteroid and the proportions described may be varied.

## Claims

1. A cosmetical method, other than a method of therapeutical treatment, for hair restoration of a patient comprising administering to an affected area of the patient a corticosteroid and tretinoin.

2. A method according to claim 1, wherein the corticosteroid comprises hydrocortisone.

3. A method according to claim 1, wherein the corticosteroid comprises betamethasone dipropionate.

4. A method according to any one of claims 1 to 3, wherein the corticosteroid and the tretinoin are applied together with a dilutant.

5. A method according to claim 4, wherein the dilutant is propylene glycol or ethanol.

6. A method according to any one of claims 1 to 5, further comprising administering a dose of Biotin to the patient.

## Patentansprüche

1. Nicht als therapeutische Behandlung gestaltetes kosmetisches Verfahren zum Haar-Wiederaufbau eines Patienten, wobei dem betroffenen Körperbereich des Patienten ein Corticosteroid und Tretinoin verabreicht werden.

2. Verfahren nach Anspruch 1, wobei das Corticosteroid Hydrocortison enthält.

3. Verfahren nach Anspruch 1, wobei das Corticosteroid Betamethason-Diproprionat enthält.

4. Verfahren nach einem der Ansprüche 1 bis 3, wobei das Corticosteroid und das Tretinoin in Verbindung mit einem Verdünnungsmittel angewandt werden.

5. Verfahren nach Anspruch 4, wobei das Verdünnungsmittel Propylenglycol oder Ethanol ist.

6. Verfahren nach einem der Ansprüche 1 bis 5, wobei dem Patienten außerdem eine Dosis Biotin verabreicht wird.

## Revendications

1. Procédé cosmétique, autre qu'un procédé de traitement thérapeutique, pour la repousse des cheveux d'un sujet, comprenant l'administration, au niveau d'une zone affectée du sujet, d'un corticostéroïde et de trétinoïne.

2. Procédé selon la revendication 1, dans lequel le corticostéroïde comprend de l'hydrocortisone.

3. Procédé selon la revendication 1, dans lequel le corticostéroïde comprend du dipropionate de bêtaméthasone.

4. Procédé selon l'une quelconque des revendications 1 à 3, dans lequel le corticostéroïde et la trétinoïne sont appliqués conjointement avec un diluant.

5. Procédé selon la revendication 4, dans lequel le diluant est du propylène glycol ou de l'éthanol.

6. Procédé selon l'une quelconque des revendications 1 à 5, comprenant également l'administration d'une dose de biotine au sujet.
